## Europäisches Patentamt
## European Patent Office
## Office européen des brevets

(11) Veröffentlichungsnummer: **0 279 461**
**A2**

(12) **EUROPÄISCHE PATENTANMELDUNG**

(21) Anmeldenummer: **88102470.7**

(22) Anmeldetag: **19.02.88**

(51) Int. Cl.4: **C09B 17/00** , G01N 33/533 , G01N 33/58 , A61K 49/00 , A61K 37/02 , G01N 33/574 , G01N 33/569

(30) Priorität: **19.02.87 DE 3705355**

(43) Veröffentlichungstag der Anmeldung:
**24.08.88 Patentblatt 88/34**

(84) Benannte Vertragsstaaten:
**AT BE CH DE FR GB IT LI LU NL SE**

(71) Anmelder: **IDT AG FUR IN VIVO DIAGNOSTIK UND THERAPIE**
**Lagerstrasse 107**
**Zürich(CH)**

(72) Erfinder: **Watzek, Carl, Dr.**
**Wichernstrasse 11**
**D-8120 Weilheim(DE)**

(74) Vertreter: **Patentanwälte Müller-Boré, Deufel, Schön, Hertel, Lewald, Otto**
**Postfach 26 02 47 Isartorplatz 6**
**D-8000 München 26(DE)**

(54) **Immunologisches Verfahren zur Bestimmung von Antigenen oder Antikörpern in biologischen Flüssigkeiten sowie Farbstoffe hierfür.**

(57) Die Erfindung betrifft ein immunologisches Verfahren zur Bestimmung von Antigenen oder Antikörpern in biologischen Flüssigkeiten sowie Farbstoffe hierfür, wobei ein Fluoreszenzfarbstoff dadurch erhalten wird, daß man Riboflavine, gelöst in Mercaptoethanol, mit Alphaliponsäure bei einem pH von etwa 8 umsetzt und nach Zugabe von Jodacetamid und Dyalisieren des Reaktionsgemisches destilliert und den Destillationsrückstand auf pH 5 bis 6 einstellt und diesen unter leichtem Erwärmen eine Reaktionslösung aus 1,4-Bis-(4-methyl-5-phenyl-2-oxazolyl)-benzol und p-Terphenyl in einem organischen Lösungsmittel, der Maleinsäureanhydrid zugesetzt ist, langsam zutropft und das Reaktionsgemisch nach mehrstündigem Stehen auf ein etwa neutrales pH einstellt und in üblicher Weise reinigt.

EP 0 279 461 A2

## Immunologisches Verfahren zur Bestimmung von Antigenen oder Antikörpern in biologischen Flüssigkeiten sowie Farbstoffe hierfür

Die Erfindung betrifft neue, analytische Farbverbindungen zur Feststellung von in vivo-und in vitro-Bindungen zwischen spezifisch bindenden Proteinen und den Substanzen, die von Proteinen spezifisch gebunden werden und deren Anwendung in Diagnostik und Therapie und der Grundlagenforschung zur Bestimmung biologischer Komponenten in niedrigsten Konzentrationbereichen.

Immunologische Verfahren beruhen auf der Fähigkeit der Antikörper, sich fest und reversibel an spezifische Antigene zu binden. Heterogenität der Antigene/Antikörper, Kreuzreaktionen mit anderen Antigenen, mit Stoffwechselprodukten, die über immunologisch reaktive Gruppen verfügen und unspezifische Faktoren beeinflussen jedoch die Bindungsreaktionen. Dazu kommen Reduzierung der Sensitivität und Spezifität durch Hilfsreagenzien und Phasentrennungen sowie Proteinschäden durch Markierungen sowie Reaktionen zwischen Beschichtungen, z.B. coated tubes, beads, beschichtete Mikrotiterplatten, usw. und Protein..

Es gibt mehr als 15 Ausdrucksformen für Ergebniswerte der Serumkonzentrationen, die gegenwärtig in der Labordiagnostik Anwendung finden.

(Mg/ml,-mg/L,-mmol/l,-mol/L,-μmol/L,-μg/dl,,μg/ml,-ng/ml, -nmol/ml,-U/L,-g/L,-mU/L,-μIE/ml,-μU/ml,-mIE/ml,-IU/ml,-u.a.m.)

Bei den immunologischen Reaktionen besteht eine nicht-lineare Abhängigkeit zwischen dem Meßsignal und der Konzentration. Deshalb wurden spezielle mathematische Funktionen entwickelt, die jeder neuen Antikörpercharge angepaßt werden müssen und unter genau festgelegten Meßbedingungen eine optimale Genauigkeit ergeben.

Die heutige Labordiagnostik mit Immunoassays, insbesondere der Enzym-Immuno-Assays, (EIA) birgt gewisse Gefahren für die Wertung der immunologischen in vivo Abläufe - also der Wirklichkeit. Sie können nur erkannt werden, wenn gleichzeitig auch die Verschiedenheiten berücksichtigt werden.

Das Hauptproblem für das eigentliche analytische Verständnis in der Immunologie ist die immunologische Spezifität, und diese ist relativ.

Die Antikörperspezifität wird bis heute mittels der Assoziations-Konstante abgegrenzt. (D.M.Weir, Handbook of experimental Immunology, Vol. 1, 16 -19, 1978, Blackwell Scientific Publication).

Obwohl ein Anti-A-Antikörper 100 bis 1000 mal besser eine Bindung mit einem A eingeht als mit einem B-Antikörper oder anderen Gruppen, gibt es aber über diese unspezifischen Bindungen keine Diskussion, sondern nur Übereinstimmung, daß der Anti-A-Komplex in Verbindung mit der A-Immunantwort eben nur spezifisch gegen A sein kann.

Solange nicht mengenmäßig, d.h. wieviel Antikörpermoleküle binden sich spezifisch an Antigenmoleküle, oder wieviel T-Zellen-Rezeptoren binden zählbar an gleichwertige Größen bestimmt werden können, solange sollten Ergebnisse, die über einfache +/- Skalen ermittelt sind, sehr vorsichtig interpretiert werden.

Die wichtigste praktische Bedeutung sollte bei jeder immunologischen Beurteilung berücksichtigt werden: Antikörper und T-Zellen sind nur effizient gegen Determinanten, die sie erkennen.

Das Immunsystem hat drei Schlüsselprobleme bei der Antigen-Erkennung zu lösen:

1 Wie unterscheidet es zwischen der großen Zahl von Fremdantigenen, um eine spezifische Antwort (response) zu gewährleisten?

2. Wie stellt es sicher, ob diese Antwort für eindringende Antigene geeignet ist und wie können diese Antigene eliminiert werden?

3. Wie wird die Immunantwort zu einer Vielzahl von Eigenantigenen vermieden?

Das Immunsystem löst seine Probleme durch Bereitstellung von Millionen von verschiedenen Immunglobulinmolekülen (Antikörper), wobei jedes über at'weichende Antigen-Bindungsstellen verfügt. Diese Immunglobulinmoleküle werden unter Millionen von verschiedenen Lymphozytenklonen verteilt, wobei jedes der Moleküle an die Stellen gebunden wird, die erkannt wurden.

Das Immunsystem ist mit einer Vielzahl unterschiedlicher Klassen und Untergruppen von Lymphozyten ausgestattet. B-Zellen produzieren Antikörper und T-Zellen entwickeln unterschiedliche Muster der zellvermittelnden Immunantwort. Glykoproteine verteilen die einzelnen Antigene in das passende Raster der Immunantwort. Sie haben die Funktion von Antigen-Erkennungsmolekülen, um bei der Verteilung von Antigenen zwischen den einzelnen Klassen zu unterscheiden.

Ein Teil der Immunglobulinmoleküle wird von Zellen und Gewebe sequestiert, und damit ist der weitere Kontakt mit Lymphozyten unterbunden, der andere Teil reicht nicht aus, um Lymphozyten zu stimulieren. Aufgrund der geringen Konzentration können daher beide Teile nicht mit den Glykoproteinen assoziieren. Andere Gruppen der Immunglobulinmoleküle snd zugänglich für Lymphozyten, assoziieren mit Glykoproteinen und aktivieren auf diese Weise die Lymphozyten.

Eindringende Fremdantigene werden von den meisten T-Zellen nur erkannt, wenn diese Fremdantigene an der Zelloberfläche mit genverschlüsselten Glykoproteinen des Histokompatibilitätskomplexes assoziieren.

Es gibt zwei Gruppen dieser Glykoproteine:

- Glykoproteine mit ausschließlicher Bedeutung und Funktion für alle kernbildenden somatischen Zellen und viraler Antigene zu zytotoxischen Zellen.
- Glykoproteine, welche in Verbindung mit Fremdantigenen durch Helfer T-Zellen erkannt werden, finden sich in dieser Art an den meisten B-Zellen, an einigen T-Zellen, Makrophagen und an speziellen antigentragenden Zellen.

Auf all diesen verschiedenen Zelloberflächen leisten die Glykoproteine in ihrer Eigenschaft als Antigen-Bindungs-Rezeptoren Hilfe für die Gruppen von Fremdantigenen, um die passenden T-Zelltypen für eine Immunantwort zu aktivieren.

Bei bestimmten Tumoren und Viren, (Retroviren) ist das Kapsid mit einer doppelten Lipidschicht umgeben, auf der in Form eines Nadelkissens eine Vielzahl unterschiedlicher Glykoproteine aufgesteckt sind.

Tauchen Viren häufig in der Blutbahn auf, dann überlasten sie das Immunsystem, eine Immunschwäche tritt zumindest zeitlich ein. Diese Überforderung des Immunsystems aktiviert die in den Zellen ruhenden anderen Erreger. Sie allein vermögen das Immunsystem dann außer Funktion zu setzen.

Die lange Inkubationszeit, die relativ geringe Infektiosität und der indirekte Nachweis von Viruspartikelchen im Rahmen einer geschwächten Abwehr spricht für ein "altes" längst bekanntes Virus.

Bis heute steht kein spezifischer mikrobiologischer, chemischer oder immunologischer Test zur Verfügung. Für die atypischen und erst beginnenden Fälle einer erworbenen Immunschwäche ist nur der intrakutane Nachweis der abgeschwächten zellulären Immunität anwendbar. Dieser Test unterliegt verschiedenen Einflüssen, wie die nicht sofortige Verarbeitung, Tageszeit der Blutentnahme, virale Infektionen, Impfreaktionen, rheumatische Erkrankungen zeigen immer eine verminderte zelluläre Immunität auf. Daher ist dieses Verfahren zur Diagnostizierung der Immunschwäche, wie auch der Antikörper-Enzym-Assay unspezifisch.

Im Vergleich mit einer in der Literatur im Zusammenhang mit Immunschwäche stehenden Virusinfektion, der Virushepatitis, ist die Unspezifität der Nachweisverfahren gleich gelagert.

Der Infektionsbeginn liegt bei der Virushepatitis B bei $2 \times 10^5$ Virus Partikel. Die untere Nachweisgrenze bei den Enzym-Immuno-Assays liegt um den Faktor 2.000 höher als die Infektionsgrenze. (Hoofnagle, et al. Civilian Disease Control, New Engl.J.Med.298,1379,1978). Die Empfindlichkeitsschwelle von 3 bis 5 ng beim Enzym-Immuno-Assay liegt hinter der Isotopentechnik. Sie ist aber ausreichend, um den untersten Normal-Serumwert zu erfasser. (R.Masseyeff, Assay of tumor associated Antigens, Quantitative Enzyme immunoassay, Scandinavian Journal of Immunology, Suppl.No.7, Vol.8, p.87, 1978).

Aufgabe der Erfindung sind somit empfindlichere Farbstoffe und ihre Herstellung und Anwendung, wobei kein Arbeiten mit gesundheitsschädlichen Substanzen erforderlich ist und in kurzer Zeit zuverlässige, nicht rechnergestützte, reproduzierbare Ergebnisse möglich sind, die ausschließlich die spezifischen in vivo-Bindungsproteine (Antigen/Antikörper) unter Berücksichtigung der qualitativen und quantitaiven Verschiedenheiten in niedrigen Konzentrationsbereichen erfassen.

Als Lösung dieser Aufgabe wurde nun gefunden, daß ein Reaktionsgemisch aus 1,4-Bis-(4-Methyl-5-Phenyl-2-oxazolyl)-benzol und p-Therphenyl(1,4-Diphenyl-benzol) in Kombination mit Maleinsäureanhydrid mit bestimmten natürlichen Farbmolekülen, die durch Umsetzung von z.B. 7,8-Dimethyl-10-(D-1′-ribityl)-isoalloxazin oder anderen bestimmten Coenzymen, wie NAD, NADH, NADPH oder Flavoproteinen mit optisch aktiven Substanzen mit Disulfidbrücken, insbesondere gewissen Coenzymen, z.B. α-Liponsäure, zu Farbstoffen führen bei denen die kritischen Abstände zwischen Absorption und Emission im Sichtbaren statt im Durchschnitt von 10 nm nur bei nicht mehr als 2 nm liegen und die beiden Ausgangsmoleküle (Farbmolekül ist gleich Donator) und Adaptermolekül (ist gleich Akzeptor) in ihren elektronischen Eigenschaften durch die Bindung nicht verändert werden. Durch die chemischen Verknüpfungen funktioneller Einheiten miteinander erhält man eine neue Funktionseinheit, die mehr leistet als die Summe ihrer Bestandteile. Es handelt sich um biofluorophore Farbstoffe.

Dies sog. Halbmoleküle werden mit anderen Syntheseteilen, die S-S Brücken (Disulfidbrücken) enthalten, verknüpft. In diesen neuen analytischen Farbverbindungen sind Bindungsstellen, wie Peptidbindungen, Disulfidbrücken, Wasserstoffbindungen, Ionenbindungen vorhanden. In der Anwendung werden durch Elektronenaufnahme Farbteile wie blau, grün, gelb und rot freigesetzt, die das Lichtabsorptionsmaximum auf 600 - 640 nm erhöhen. Dies ermöglicht die gezielte Phototherapie, insbesondere zur Einführung monoclonaler Antikörper und anderer Therapeutika.

Die biologische Signifikanz der neuen Fluoreszenzfarbstoffe liefern die molekulare Basis der Immunogenität und anitgene Spezifität ohne zusätzlich Trägermoleküle, z.B. Einweißkörper.

Moleküle kurzkettiger Peptide in Form von Rezeptoren fungieren als Auslöser oder Begleiter neoplastischer Prozesse und unterstreichen eine neue Gewichtung in der Analytik. Tumorzellen produzieren Faktoren, die ihr eigenes Wachstum stimulieren. Ein Tetradecapeptid - Bombesin - reagiert mit spezifischen Rezeptoren der Tumorzellen, - als zellständige Antikörper im Test anwendbar - die von der Zelle produziert, aber nicht nennenswert sezerniert werden, kann nur in niedrigen Konzentrationsbereichen (femto-, attomol) nachgewiesen werden.

Für alle Verfahren, bei denen neue analytische Farbverbindungen eingesetzt werden, ist die spezifische molekulare Verbindung ausschlaggebend.

Die neuen analytischen Farbverbindungen können ohne Trägersubstanzen, z.B. Albumine (RSA, BSA, HSA) einem Tier in physiologischer Kochsalzlösung oder Puffer als Träger injiziert werden, welche dann durch Bildung von fluoreszenzmarkierten Antikörpern gegen diese Farbverbindungen reagieren. Spezifische Proteine, wie Antigene, Tumorantigene, virale Antigene, sowie alle Arten niedermolekularer oder hochmolekularer Substanzen können mit den neuen analytischen Farbverbindungen verknüpft und , einem Tier injiziert werden, welches dann in vivo fluoreszenzmarkierte spezifische Antikörper bildet. Dieselbe Verfahrensweise gilt auch für Zellkulturen oder für Proteine, die von einem reaktiven oder autonomen, exzessiv proliferierten Plasma-Zellklon gebildet werden. Die in vivo Markierung kann auch zur Produktion von monoclonalen Antikörpern eingesetzt werden.

Diese in vivo-fluoreszenzmarkierten spezifischen polyklonalen oder monoklonalen Antikörper für die Durchführung von quantitativen und qualitativen Bestimmungsverfahren gemäß der Erfindung werden entweder als Testkit, Einzel-Reaktionslösung, als beschichtete Mikrotiterplatte, beschichtete Röhren, Objektträger, Streifen oder gestanzte Filterpapier plättchen, als Gele, Harze usw., also in an sich üblicher Form, verwendet.

Ein Testkit besteht hauptsächlich aus:

1) der bestimmten Menge eines Farbstoffkopplungsproduktes, wahlweise einer bestimmten Menge der Komponente Antigen/Antikörper in unlöslicher Form,

2) einer bestimmten Menge eines Reaktionsgemisches zur Trennung der freien ungebundenen Farbstoffmenge,

3) einer bestimmten Menge Pufferlösung.

Die in vivo-fluoreszenzmarkierten polyklonalen oder monokonalen Antokörper verstärken die körpereigene Abwehr in der Tumortherapie und können gezielt als Träger spezifischer Therapeutika eingesetzt werden.

Die vorliegende Erfindung bezieht sich auf die Verwendung von Biomolekülen aus optisch aktiven Substanzen, die zur Eiweißsynthese verwendet werden und die in der Lage sind, die Schwingungsebene von linear polarisiertem Licht beidseitig zu drehen.

Diese chemisch synthetisierten Kopplungsmoleküle können mühelos in Gruppen genetisch identischer Zellen, Klone oder Mikroorganismen eingebaut werden.

Gleichzeitig können diese Adaptermoleküle an Di-, Polysaccharide und Glykoproteine gebunden werden, die in Nährmedien dann durch Fermentation von den Bakterien oder anderen Kulturen aufgenommen werden und als sichtbare neutrale Farbstoffe leicht zu identifizieren sind. Die Verwendung solcherart hergestellter biofluorophorer Farbstoffe ermöglicht die einfache Herstellung von markierten Proteinen, Bakterien, Viren, Hormonen und anderen biologischen Substanzen. Diese Bio-Kopplungsmoleküle können für Applikationen in der Biotechnologie, Pharmakologie oder in industriellen Herstellungsprozessen verwendet werden.

Erfindungsgemäß ist es auch möglich, lebende Zellen mit den neutralen fluoreszierenden Adaptermolekülen in einer Konzentration von $10^{-12}$ und höher darzustellen, ohne daß eine Zerstörung der Vitalzellen eintritt. Bei Experimenten trat nach Stunden (6-8 Std.) weder eine Zellzerstörung, noch eine Photobleichung ein.

Die Erfindung wird durch die folgenden Beispiele näher erläutert.

Beispiel 1

500 mg eines Flavoproteins, vorzugsweise Riboflavin-5'-monophosphat-Natriumsalz, wurden in 10 ml 2-Mercaptoäthanol gelöst. Nach Lösung wurden 500 μl 0,1 M α-Liponsäure (205.34 mg/10 ml phys. NaCl) zugegeben.

Das Reaktionsgemisch wurde durch Zugabe von 1M Tris-(Hydroxymethyl-1,3-Propandiol) Puffer auf pH 8,0 eingestellt und 4 Std. bei Raumtemperatur leicht gerührt.

Danach wurden dem Reaktionsgemisch 500 μl 0,2 M Jodacetamid zugeführt. Mit geringer Zugabe von 1M Tris-Puffer wurde der pH-Wert auf 8,0 gehalten. Nach 60 Min. wurde das Gemisch gegen Aqua bidest 16 Std./4°C dialysiert. Danach wurde das Gemisch bei 5000 Upm/20 Min. zentrifugiert, der Überstand wurde verworfen. Niederschlag wurde in 10 ml PBS-Puffer, pH 7,2 unter leichtem Rühren gelöst. Mit der einfachen Destillation ohne Rücklauf wurden überschüssige Lösungsmittelreste abgedampft (kondensiert). Das von Lösungsmitteln gereinigte Destillat wurde auf pH-5,0 eingestellt. Es enthält Farbmoleküle mit katalytischer Aktivität. Diese natürlichen neutrophilen Farbmoleküle verfügen über Peptid-und Disulfid-Brücken.

Die Farbmoleküle aus Riboflavinen können aus den Farbmolekülen von anderen Coenzymen wie NAD, NADH, NADP sowie ande ren optisch aktiven Substanzen, welche bestimmte Bindungsmerkmale enthalten, ersetzt werden.

## Beispiel 2

4,2 ml Cyclohexan und 4,4 ml Dioxan wurden in je 500 ml gereinigtem Äthanol gelöst.

In 500 ml Reaktionsgemisch wurden unter ständigem Rühren 0,0115 g p-Terphenyl-(1,4-diphenyl)-benzol gelöst.

In 500 ml Reaktionsgemisch wurden unter ständigem Rühren 0,01962 g 1,4-Bis-(4-methyl-5-phenyl-2-oxazolyl)-benzol gelöst.

Unter ständigem Rühren wurden 98,06 mg Maleinsäureanhydrid in 10 ml gereinigtem Äthanol gelöst. Je 1 ml dieser 0,1 M Maleinsäureanhydridlösung wurden den Reaktionsgemischen unter Rühren zugegeben.

Für Kopplungszwecke kann wie folgt verfahren werden: Wird nur 1 Antiserum (Antikörper/Antigen versch. Arten) in den zu erstellenden Testsystemen benötigt, kann die Reaktionslösung I aus Beispiel 3 verwendet werden.

Werden mehr Antiseren, z.B. HIV I, HIV II, HIV III, (Antikörper gegen human immundefiency virus) oder Immunglobuline der Klassen A, E.D.M, G und deren Untergruppen, oder virale Erkennungsproteine, wird die Reaktionslösung II benötigt. Die Zugabe von 1 ml 0,1 M Maleinsäureanhydrid bewirkt eine reversible Blockierung von unspezifischen Aminogruppen.

## Beispiel 3

Verknüpfung von Farbmolekülen (Donator) mit der Löschersubstanz (Akzeptor) zur Reduzierung der intramolekularen Triplett-Triplett-Energieübergangs.

5 ml Farbmoleküllösung wurde im Kolben leicht erwärmt (45°C). Unter ständigem Rühren wurden 500 ml einer Reaktionslösung aus Beispiel 2 langsam eingetropft. Das Reaktionsgemisch wurde im Kolben verschlossen und 4 Std. bei Raumtemperatur stehen gelassen. Danach wurde das Gemisch auf pH-Wert -7,2 eingestellt und gegen 0,1 M PBS-Puffer 2 Std. dialysiert und anschließend steril gefiltert. Mit der zweiten 500 ml Reaktionslösung aus Beispiel 2 wurde ebenso verfahren. Beide Reaktionslösungen können unter leichtem Rühren zusammengemischt werden. Diese Mischung ergibt Reaktionslösung I. Für die Reaktionslösung II bleiben die zwei Reaktionslösungen aus Beispiel 2 getrennt. Diese zwei Mischungen ergeben die Reaktionslösung II. Diese zwei Lösungen werden erst nach der Kopplung miteinander vermischt.

## Beispiel 4

Kopplung von Proteinen an biofluorophore Farbstoffe zum Nachweis und zur Bestimmung von Farbstoff-/Protein-Kopplungsprodukten der molaren Bindungen in biologischen Flüssigkeiten.

5 ml Farbstoff-Lösung wurden mit 0,05 M PBS-Puffer auf 40 ml aufgefüllt und im Eisbad unter Rühren mit 0,3 M Boratpuffer auf pH-8,5 eingestellt.

Die Proteinkonzentration sollte maximal 1,5 mg/ml betragen. Antiseren mit hoher Proteinkonzentration wurden mit Na$_2$SO$_4$ 1 Std. gerührt, der entstandene Niederschlag wurde abzentrifugiert, gewaschen und wieder auf pH-8,5 eingestellt.

Zu der gekühlten biofluorophoren Farbstofflösung wurden 5 bis 10 ml der oben aufbereiteten Proteinlösung langsam unter Rühren eingetropft. Das Reaktionsgemisch wurde 16 Std. bei 4°C langsam gerührt, mit 0,1 m PBS-Puffer auf pH-7,2 eingestellt und gegen Aqua bidest. 2 Std. dialysiert. Danach Sterilfiltration.

## Beispiel 5

Herstellung eines Konjugates für die direkte und indirekte Immunfluoreszenztechnik in Cyto-Histochemie und Pathologie.

5 ml Farbstoff-Lösung wurden mit 0,15 M $Na_2HPO_4$ $2H_2O$ auf 20 ml aufgefüllt und auf pH-9,0 eingestellt. Unter ständigem Rühren wurde bei Raumtemperatur die Proteinlösung (0,5 mg/ml) zugegeben. Farbstoffzugabe bis max. 1,5 mg/ml Protein.

Nach 60 Minuten wurde der pH-wert auf 7,2 eingestellt. Danach Konjugat-Reinigung mittels Ionenaustausch-Chromatographie. Säule 2 × 20 cm/10 g Cellulose, 10 ml Probe, Durchfluß Vol. 300-400 ml.

## Einfache Konujugation

40 $\mu$g getrockneter Farbstoff wurden unter ständigem Rühren einer NaCl-Proteinlösung zugegeben und der pH-Wert der Reaktionslösung auf pH-9,5 eingestellt. Nach einer Stunde freien Farbstoff über eine Säule mit Sephadex-G-25 entfernen.

## Beispiel 6

Kopplung von Farbstoff-/Proteinlösungen an Festkörperphasen, wie Mikrotiterplatten, Polystyren beads, glassbeads, Suspensionsgele, Coated tube (beschichtete Röhrchen).

1 g getrocknetes Gel = 2,5 bis 3 ml Gel

Je 1 g getrocknetes Gel wurde mit 200 ml $10^{-3}$ HCl-Boratpuffer pH-8,4 gewaschen.

In einem kleinen Gefäß wurden die Gelsuspension und Farbstoff-/Proteinkopplungsprodukt (Beispiel 4) 24 Std. bei Raumtemperatur unter ständigem Rühren inkubiert. Bei diesem Reaktionsgemisch sollte die Gelsuspension relativ wenig Flüssigkeit aufweisen, sonst tritt eine zu hohe Verdünnung der Proteinlösung ein (Säulenpackung eben bedeckt). Das Reaktionsgemisch wurde bei 1500 Upm zentrifugiert und der Überstand für die OD-Messung aufbewahrt.

Das Gel wurde mit der 3-fachen Menge 1 m Tris-Puffer auf pH-8,0 eingestellt und 1 Std. bei Raumtemperatur unter Rüh ren inkubiert, die Suspension zentrifugiert und der Überstand verworfen. Der Rückstand wurde mit dem 5-fachen Volumen 0,1 M PBS-Puffer gewaschen, über eine Sephadex G 25 Säule gereinigt. Die gereinigte Lösung wurde in PBS-Puffer + 0,02 % Na-Azid aufbewahrt.

## Beispiel 7

Kopplung von biofluorophoren Farbstoffen an Proteine zur Herstellung von supravitalen Fluoreszenzreagenzien zum Studium an den lebenden Zellen. Proteinkonzentration: 0,2 mg/ml - 1,0 mg/ml

Pufferlösung: 0,05 M Boratpuffer, pH-9,5

Die Proteinlösungen wurden gegen 0,05 M Boratpuffer, pH-9,5 über Nacht bei 4°C dialysiert.

10 ml Farbstofflösung (Beispiel 3) wurden mit 100 ml Boratpuffer unter Rühren vermischt. Der Dialyseschlauch mit dem Protein in die Farbstoff-/Pufferlösung eingehängt und 12 Std. im Dunkeln inkubiert (Gefäß mit Alufolie umwickeln oder im Dunkelraum dialysieren). Das markierte Protein wurde im Dialyseschlauch belassen und gegen ein großes Volumen 0,1 M PBS-Puffer pH-7,2 dialysiert. Der Puffer wurde mehrmals gewechselt. Die Dialyse erfolgte im Dunkeln bei 4°C/12 Std. Die PD-Säulen (Sephadex G-25) wurden mit 10 ml PBS-Puffer 0,1 M equilibriert. Maximal wurden 2,5 ml markiertes Protein aufgetragen. Die Proteinlösung wurde ganz einlaufen gelassen. Auf die Säule wurden 3 ml PBS-Puffer gegeben und das Eluat aufgefangen. Es erfolgte eine klare Trennung des freien Farbstoffes vom markierten Protein. Anschließend erfolgte Sterilfiltration.

Mittels Mikroinjektions-Vorrichtung (Zeiss) wurde diese Farbstoff-/Proteinlösung in die lebende Zelle injiziert.

Beispiel 8

Herstellung einer Beschichtungslösung zur Beschichtung von z.B. Mikrotiterplatten, Röhrchen, Strips mit unmarkierten oder markierten Antiseren.

Diesem Verfahren liegen 0,5 ml Antiserum (Antiserum = Antigen, Antikörper oder Hapten) zugrunde, bei einem Gesamtvolumen von 35 Liter Beschichtungslösung.

Reagenzien:

Puffer:

PBS 0,01 M / pH-6,0 / 20 Lt.

PB 1,0 M / pH-9,5 / 20 Lt.

wässrige Glutaraldehydlösung 0,1 M / 107,5 ml

Natriumchloridlösung 0,13 M / 50 ml

Natriumazidlösung 6 g / 25 ml Aqua bidest

Ascorbinsäurelösung 1,13 g / 25 ml Aqua bidest

Antiserum 0,5 ml

1) 750 ml PBS-Puffer, pH-6,0/0,01 M wurden in einem Reaktionsgefäß auf 37°C erwärmt und auf dieser Temperatur gehalten.

2) 0,5 ml Antiserumpräzipitat wurden in üblicher Weise 1 Std. bei 4°C zentrifugiert (1000 Upm). Überstand wurde vorsichtig dekantiert und in 0,5molar. frischem PBS-Puffer, pH-6,0 gelöst und gerührt bis zum klar.werden. 750 ml des erwärmten Puffers wurden unter Rühren langsam dazugegeben. Die Lösung wurde konstant auf 37°C gehalten.

3) 107,5 ml frische 0,1 M Glutaraldehydlösung wurden auf 37°C erwärmt und unter Rühren obiger Lösung zugegeben und bei 37°C 20 Min. inkubiert. Dann wurde die Lösung von der Wärmeplatte genommen.

4) 750 ml eines 1,0 M PB-Puffer, pH-8,5 wurden der Lösung aus 3) zugegeben und unter Rühren vermischt. Zu dieser Reaktionslösung wurden 50 ml einer 0,2 M Natriumchlordlösung zugegeben und weiter 30 Min. bei Raumtemperatur gerührt. Anschließend wurden 25 ml einer 4,5 %igen Ascorbinlösung und 25 ml einer 24 %igen Natriumazidlösung unter Rühren zupipettiert.

5) 16,65 ml PBS-Puffer wurden in einem gesonderten Gefäß mit 16,65 ml PB-Puffer vermischt und auf pH-8,5 ± 0,2 eingestellt.

6) Zu diesem 33,3 ml. Puffergemisch wurde die polymerisierte Lösung zugegeben und unter leichtem Rühren gut vermischt.

7) Mikrotiterplatten, Röhrchen, Strips wurden überprüft und in jede Vertiefung 50 µl/100 µl Beschichtungslösung pipettiert, mit Parafilm abgedeckt und über Nacht bei Raumtemperatur inkubiert.

8) Platten, Röhrchen, etc. wurden vorsichtig abgesaugt und mit Tris-Puffer 3× gewaschen, getrocknet und bis zum Gebrauch in Folien eingeschweißt.


Beispiel 9

Festphasen - (solid phase) Fluoreszenzimmunoassay

Die mit Antiseren oder inaktivierten Viren beschichteten Vertiefungen der Mikrotiterplatten, Röhrchen oder Strips wurden mit 250 µl Molkenalbumin abgesättigt (30 Min. bei 37°C) und einmal mit PBS-Puffer gewaschen. 200 µl eines fluoreszenzmarkierten Antikörpers (Beispiel 4) wurden in die Vertiefungen, bzw. Röhrchen pipettiert. Platten bzw. Röhrchen wurden bei leichtem Schütteln 1 Std. bei 37°C inkubiert.

Nach der Inkubation wurden die Platten oder Röhrchen 3× mit PBS-Puffer gewaschen. Nach Abtupfen wurde in jede Vertiefung oder Röhrchen 100 µl Stopplösung pipettiert. Nach 2 Min. wurde mit Aqua bidest gewaschen, abgetupft und im Photometer oder Fluorometer oder Spetralphotometer entweder bei 345 nm oder 366 nm gemessen. Berechnung:

Proteinkonzentration im Serum

Die Proteinvergleichslösung zeigt bei 345 nm und 366 nm eine Extinktion von X, die einer Konzentration von XY femtomol Protein/mol entspricht.

$$\frac{E_{Probe}}{E_{Vergleichslösung}} = x \text{ mol Protein/ml Vergleichslösung}$$
$$\text{mol Protein/ml Probe}$$

$$\text{Faktor: } \frac{\text{mol Protein/ml Vergleichslösung}}{E_{Vergleichslösung}}$$

Beispiel 10

Herstellung eines Spot-Testes zur Charakterisierung von Seren, Antikörpern/Antigene.

Mit einer Einmal-Mikropipette wurden 0,5 µl eines Antiserums (Konzentration 0,01 - 1,0 mg/ml) auf Nitrocellulosestreifen getüpfelt.

Dabei darf die Nitrocellulose nicht beschädigt werden. Der Tropfen muß eintrocknen. Die Nitrocellulose wurde mit einer Blockierungslösung (3 %iges hochgereinigtes Molkenalbumin in PBS, pH-7,2) abgesättigt. Nach Absättigung ist der Mikrocellulosestreifen nicht mehr aufnahmefähig für Fremdproteine. Das Tragen von Gummihandschuhen wird empfohlen.

Fluoreszenzmarkierte Antikörper (Beispiel 4) und fluoreszenzmarkierte Protein-Molekulargewichts-Standards (Beispiel 11) in Endkonzentrationen von 100 - 10 µg/ml und 0,5 - 0,1 µg/µl wurden mit dem Nitrocellulosestreifen 45 Minuten bei 37°C unter leichtem Schütteln inkubiert. Nach der Inkubation wurde der Nitrocellulosestreifen ausgiebig mt PBS-Puffer gewaschen (Spritzflasche).

Die Reaktion wurde mit 0.1 M NaOH gestoppt. Nach 2 Minuten wurde mit Aqua bidest abgewaschen. Die Streifen sind im Dunkeln, bzw. abgedeckt aufzubewahren.

Beispiel 11

Herstellung von Protein-Molekulargewichtsstandards.

1) Hochmolekulargewichtsstandard mit einer Endkonzentration von 0.5 µg/µl für MG-Bereiche 17.000 - 2.000.000.

Mit je 500µl Aqua bidest wurden die lyophilisierten Protein-Molekulargewichts-Standards (PMGS) in einem Becherglas unter sanftem Rühren langsam aufgelöst. Nach Auflösung wurde das Rühren eingestellt. Das Becherglas wurde bei 65°C/ 15 Min. in ein Wasserbad gestellt.

Es wurden 475 µl einer 8 M Carbamidlösung in 0.05 M Tris/HCl-Puffer, pH-8.0 hergestellt und langsam in das Becherglas pipettiert. (480.48 g Carbamid, 6.05 g Tris im kochenden Wasser gelöst. Nach Erkaltung auf pH 8.0 mit konzentrierte HCl eingestellt und zu einem Gesamtvolumen von 1000 ml aufgefüllt.)

Herstellung der Kopplungslösung

50 ml Kopplungslösung (Beispiel 3) wurden mit 5ml Glycerin unter Rühren gut gemischt.

25 µl obiger Reaktionslösung wurden zu 475 µl Carbamidlösung zugegeben und langsam bei Raumtemperatur 3 Stunden gerührt.

PMGS wurden in Aliquots von 50 - 100 µl eingefroren.

2) Nieder-MG-Standard mit einer Endkonzentration von 0.1 µg/µl für MG-Bereiche 1000 - 17 000.

250 µl 10%ige Natriumdodecylsulfatlösung, 500 µl 10%ige Dithiothreitollösung und 250 µl 0.05 M Tris/HCl-Puffer wurden unter Zugabe von 25 µl 5%iger Glycerinlösung unter Rühren gemischt.

Die lyophilisierten PMGS wurden im Becherglas mit 250µl Puffergemisch unter Rühren aufgelöst, das Reaktionsgemisch im Wasserbad 5 Min. auf 100°C unter Rühren erhitzt und nach Abkühlung auf ca. 37°C wurden 25 µl Kopplungslösung zugegeben und es wurde 3 Stunden bei Raumtemperatur gerührt. Die PMGS wurden in Aliquots von 50 - 100 µl eingefroren.

Beispiel 12

Herstellung einer Kopplungslösung zur Markierung von viralen Proteinen, Immunglobulinen, Zellkulturen, infektiösen Zellen, Viren und anderen Mikroorganismen, Klonen, Aszitesflüssigkeiten, monoclonalen Antikörpern, Antigenen aller Art, Rezeptoren, Haarzellen und Cilien für in vivo-Diagnostik und -Therpie.

1) 100 ml der Kopplungslösung (Beispiel 3) wurden redestilliert.

2) 50 ml Kopplungslösung wurden auf 37°C erwärmt und auf pH 7.2 eingestellt.

3) 5 - 10 ml einer vorbereiteten Antigen-oder Antikörperlösung wurden langsam der erwärmten Kopplungslösung zupipettiert. Das Reaktionsgemisch wurde mit frischem Boratpuffer 0.1 M auf pH 5.0 eingestellt und 12 Stunden bei 4°C gerührt.

4) 100 mg S-Acetylmercaptosuccinic-anhydrid wurden in 5 ml PBS-Puffer gelöst, dem Reaktionsgemisch unter Rühren langsam zugegeben und 5 Stunden bei Raumtemperatur inkubiert.

5) das Reaktionsgemisch wurde 10 Min. bei 30.000 Upm zentrifugiert und der Übersand steril gefiltert.

Beispiel 13

Aufgrund der hohen Antigenvariabilität der Erreger, wie Retroviren (LAV-HTLV-III,-HIV-III) wird ein Immunprofil erläutert, das außer dem üblichen Antikörpernachweis auch die spezifische Bestimmung des Immunschwächegrades und des Infektionsgrads der beteiligten Erreger ermöglicht.

Viren, insbesondere HIV-dringen über einen speziellen Rezeptor in die Zellen ein und passieren auch die BLUT-HIRN-Schranke.

Astrozyten, Macrophagen, Phagocyten, Haarzellen und Cilien verfügen über eine Anzahl von speziellen Oberflächenrezeptoren, die funktionell an die Zellen gebunden sind. Das auffallendste Charakteristikum dieser Rezeptoren ist die Erkennung von Antikörpermolekülen. Eines der Hauptmerkmale zum Schutze gegen Fremdantigene ist die Bindung an der Rezeptoroberfläche, um eine Hülle zu bilden, die von Macrophagen und polymorphen Leukozyten durch den Rezeptor erkannt wird.

Diese Rezeptorbindung induziert die Zellen zur Bildung phayocytärer Vacuolen, um Fremdpartikel wie Antigene zu verschlingen und aufzulösen.

Der neue IDT-(immun-deficiency-test) Kit für spezifische Diagnostik und Therapie weist 4 wichtige Merkmale, modifiziert und zusammengefaßt in einer Mikrotiterplatte (Immunprofil) auf.

1 Immunglobulinbestimmungen zum Nachweis der Beteiligung an und zur Abgradierung der Immunschwäche,

2 der sog. Western-Blot, modifiziert und in die Mikrotiterplatte integriert zum Nachweis viraler Proteine unter Mitführung von Protein-Molekulargeichts-Standards (PMGS).

3 spezifischer HIV-III-Test

4 Erregernachweis (16 Erreger die bei der AIDS-Erkrankung eine wichtige Rolle spielen) zur Festellung des Infektionsgrades.

Der IDT-Kit (mit oder ohne Immunprofil) kann variabel für zahlreiche IMMUN-Disorders,-(Immunstörungen) eingesetzt werden, z.B.

IDT-Kit/AU = Autoimmunkrankheiten

IDT-Kit/All = Allergien

IDT-Kit/M = Maligne Erkrankungen

Der Test gestattet eine photometrische, spektralfluorometrische oder visuelle Direktablesung in niedrigsten Konzentrationsbereichen ohne zusätzliche statistische Auswertung und Empfindlichkeitslimitierung. Es wird in diesem Testverfahren ausschließlich nur die echte molekulare Bindung zwischen Antigen und Antikörper bestimmt. Aufgrund der hohen Photostabilität können die einzelnen Endresultate weit über ein Jahr als Dokumentation bei Wiederholungsbestimmungen verglichen werden.

Herstellung eines Festphasen-Immunprofils

1) Bereitstellung von beschichteten Mikrotiterplatten, Röhrchen, Strips, Cellulose-/Papierstreifen, Objektträgern, Plastikugeln, Latex-Partikel usw. Es wird die Strip-Platte mit auswechselbaren Streifen empfohlen.

2) Aufteilung der Mikrotiterplatte in Strips.

```
Reihe/Strip  1,A-H  Immunglobuline A,D,E,G,M und PMG-
                    Standards
             2,A-H  6 Näpfe für Wiederholungsbestimmungen
Reihe/Strip  3,A-H  virale Proteine, GP 160, GP 110, P 68,
                    GP 41, P 40, P 34, P 25, P 18, P 13
             4,A-H  HIV-III
             5,A-H  HIV-III - Bestimmungen
             6,A-H
Reihe/Strip  7,A-H  monoklonale Antikprper
             8,A-H  Candidamykosen, Chlamydia, Cytomegalie
             9,A-H  Epstein-Barr, Entamöben, Hepatitis A,B,C
            10,A-H  Herpes 1,2, 1+2 Listerien, R.S.V.(Res-
            11,A-H  piratory Syncytial virus),Toxoplasmose,
            12,A-H  Pneumocystis carinii, Strongyloides
                    stercoralis. und negative und positive
                    Kontrollen.
```

Für 1 Testbesteck/10 Immunprofile (A 96 Bestimmungen) wurden folgende Reagenzien benötigt:

$5 \times 5$ ml fluoreszenzmarkiertes Anti-A, Anti-D, Anti-E, Anti-G, Anti-M

$15 \times 5$ ml fluoreszenzmarkierte virale Proteine/Protein-Molekulargewichts-Standards-PMGS 150,900,400, 175, 190, 160, 110, 68, 55, 41, 40, 34, 25, 18, 13

$1 \times 10$ ml Anti-HIV-III-Antikörper, fluoreszenzmarkiert,

$16 \times 0,5$ ml fluoreszenzmarkierte Antikörper für die unter 2 aufgeführten 16 Erreger

5 ml negative Kontrolle, 5 ml positive Kontrolle

15 ml 1M NaOH

5 ml hochgereinigtes Molkenalbumin

1000 ml Pufferwaschlösung (in Granulatform)

Testablauf:

1) in jedes beschichtete Näpfchen wurden 50 - 100 $\mu$l Probe pipettiert. Die Platte wurde abgedeckt und 30 Min./40°C inkubiert.

2) 50 $\mu$l Molkenalbumin wurden in die mit Probe gefüllten Näpfchen zugegeben und nochmals 15 Min./40°C inkubiert und $1\times$ mit Puffer gewaschen.

3) Zugabe von 50 $\mu$l fluoreszenzmarkierten Antiserum, Inkubation 60 Min/37°C oder 45 Min./40°C und $2\times$ mit Puffer gewaschen.

4) 50 µl Stopplösung (NaOH) wurden zupipettiert, nach 2 Min. erfolgt die Messung. Ein PMGS kann mitgeführt werden.

Messung bei 354nm bis 366 nm. Berechnung der molekularen Bindung wie in Beispiel 9.

## Beispiel 14

Herstellung eines Flüssigphasen-Fluoreszenz-Immunoassay zum Nachweis und zur Bestimmung von Tumormarkern, ins. Tumorpromotern oder Agenzien mit potentiellen Möglichkeiten bösartige Tumore zu verursachen.

1) 10 ml Kopplungslösung (Beispiel 3) wurden mit 0.1M PBS-Puffer pH-8.5 auf 38 ml aufgefüllt und unter Rühren vermischt.

2) 2 ml einer $10^{-4}$ molaren D-myo-Inositol-triphosphat-Lösung wurde unter Rühren langsam zu Lösung 1 eingetropft und 3 Stunden bei Raumtemperatur inkubiert.

3) Mit 5 ml der Reaktionslösung 1 wurde 1 mg Bombesin unter Rühren aufgelöst und unter weiterem Rühren bei Einhaltung des konstanten pH-Wertes von 8.5 dem Reaktionsgemisch wieder langsam zugeführt und bei Raumtemperatur 4 Std. inkubiert.

Der pH-Wert wurde auf 7.2 eingestellt und dann wurde gefiltert.

Testverfahren:

1) in 1 Reagenzröhrchen wurden 200 µl unbekannte Probe, 50 µl sterile D-myo-Inosit/Bombesinlösung und 500 µl 0.1 M PBS-Puffer, pH 7,2 pipettiert und unter Schütteln 1 Std. bei Reampteratur inkubiert. 1 Reagenzröhrchen wurde statt mit 50 µl Bombesinlsöung mit 50 µl PMGS-Standard gefüllt und weiter so behandelt wie die unbekannte Probe.

2) in das Reagenzröhrchen wurden 50 µl Enteiweißungslösung pipettiert, das Röhrchen wurde kräftig geschüttelt und bei 3000 Upm/15 Min. zentrifugiert.

3) Der Überstand -500 µl - wurde sofort vorsichtig dekantiert und im Wasserbad 5 Min./45°C inkubiert und danach auf Raumtemperatur abgekühlt.

4) 400 µl PBS-Puffer wurden zugegeben und es wurde kräftig geschüttelt. 100 µl 1.5 M NaOH wurden zupipettiert, es wurde kurz geschüttelt und sofort gemessen.

Messung bei 354nm bis 366 nm. Berechnung der molekularen Bindung wie im Beispiel 9.

Testkitinhalt:

1 Fläschchen/5ml D-myo-Inosit-Triphosphat/Bombesin, pH-7.2
1 Fläschchen/5ml PMGS-Standard 0,06 µg Farbstoff/ml
2 Fläschchen PBS-Granulat (f.pH 7,2)
1 Fläschchen/5 ml TCA 5N
1 Fläschchen/5 ml HCl 2N
1 Fläschchen/10 ml 1.5 M NaOH

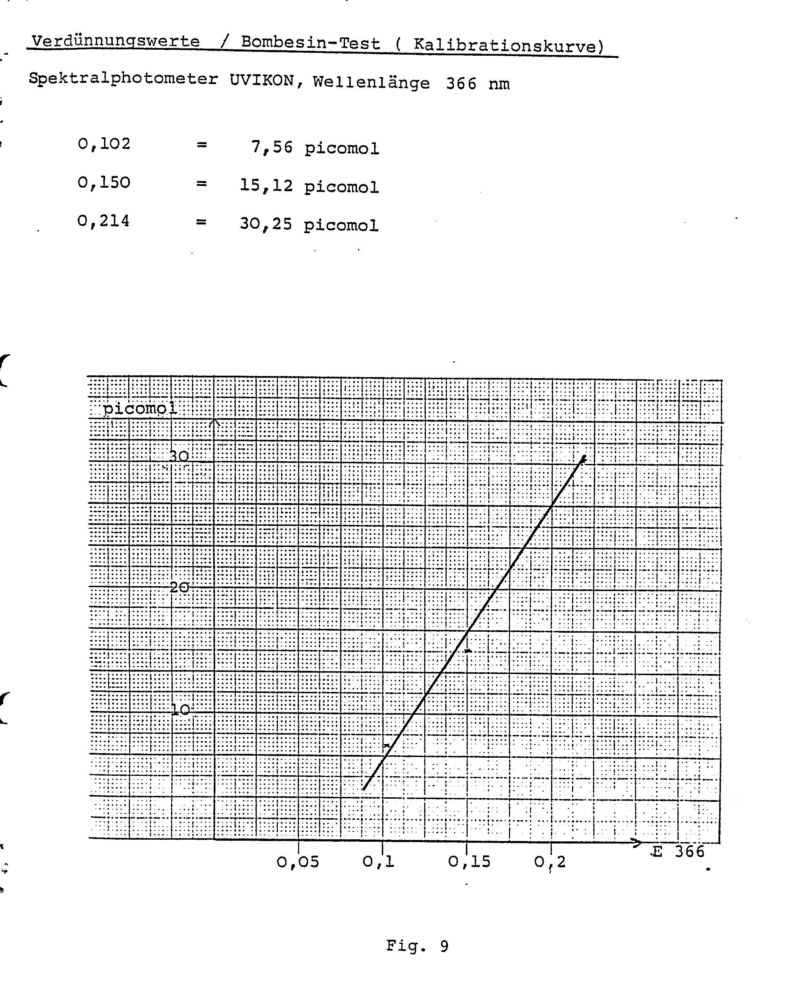

Fig. 9 zeigt die Kalibrationskurve des Bombesintests.

## Beispiel 15

Herstellung eines Immunomarkers,der zur Ausbildung humoraler und zellvermittelter Immunität führt, mit Produkten einer Immunantwort spezifisch reagieren kann und als Träger für Therapeutika, z.B. Neurotransmitter besonders geeignet ist, da der biofluorophore Immunomarker die Blut-Hirnschranke passiert.

25 µg biofluorophorer Farbstoff (Beispiel 3) wurden in 5 ml steriler physiologischer Kochsalzlösung gelöst.

Alle verwendeten Glaswaren wurden 2 Std./180°C sterilisiert. Nach Auflösung wurde das Reaktionsgemisch mit PBS-Puffer auf pH-7.2 eingestellt, steril gefiltert und steril in Aliquots von 0.05/0.5 ml verschlossen im Kühlschrank aufbewahrt.

Herstellung einer Phenol-Kontroll-Lösung.

4,97 g Phenol,puriss. wurden mit steriler physiologischer Kochsalzlösung soweit aufgefüllt, bis eine 0,25 Gew.-%ige Phenolkontroll-Lösung entstand. Nach Auflösung und Sterilfiltration wurde die Phenolkontroll-Lösung in sterilen Gefäßen 48 Std. bei Raumtemperatur aufbewahrt. Für den intrakutanen Test wurden 3 Kaninchen vorbereitet.

K 1 = Normaltier ohne Injektion

K 2 = 0.05 ml Immunomarker - nach 24 Std. keine Reaktion

K 3 = 0.05 ml Phenolkontroll-Lösung - nach 10 Min. Erythemareaktion

Die 1. Blutentnahme (n.30 Tg) zeigte in der Elektrophorese einen leichten Anstieg eines Immunglobulin G, der sich bei der 2. Blutentnahme (45. Tg) deutlich ausgeprägter zeigte. Im Gegensatz dazu zeigte sich beim Kontrolltier (K 1) keine Immunglobulin-Bande.

Das Blut des immunisierten Tieres (K 2) aus beiden Entnahmen wurde gepoolt und 30 Min. bei 2000 Upm zentrifugiert.

## Isolierung des reinen IgG mit Natriumsulfat

Das Serum wurde gegen 0,1 mol/l Phosphatpuffer, pH-8,0, 3 Std. dialysiert.

50 ml einer gesättigten Natriumsulfatlösung ($Na_2SO_4$) wurden zu 100 ml Serum zugegeben und unter Rühren eroigte die erste Präzipitation. Das Gemisch wurde 20 Min. bei 2500 Upm zentrifugiert, der Überstand wurde abgesaugt.

Der Niederschlag wurde in 25 ml gesättigter $Na_2SO_4$-Lösung aufgelöst und 20 Min. bei 2500 Upm zentrifugiert.

Die Präzipitationsschritte wurden 4 bis 5 × wiederholt. Das letzte Präzipitat wurde in 10 - 15 ml PBS-Puffer, pH-7,2 aufgenommen. Das vorgereinigte Immunglobulin wurde über eine Sephadex-Säule gereinigt. Das Eluat wurde abschließend über 1 mg lyophilisiertes Stroma * nochmals gereinigt.

Damit wurden Reste von Sphingosin, Fettsäuren, Glucose, Galactose, Glucosamin, Sialinsäure, Fucose und große Teile von Kohlenhydrate, die unspezifische Bindungen verursachen, entfernt.

Die Ausbeute ergab ein hochgereinigtes, in vivo fluoreszenzmarkiertes Immunglobulin G.

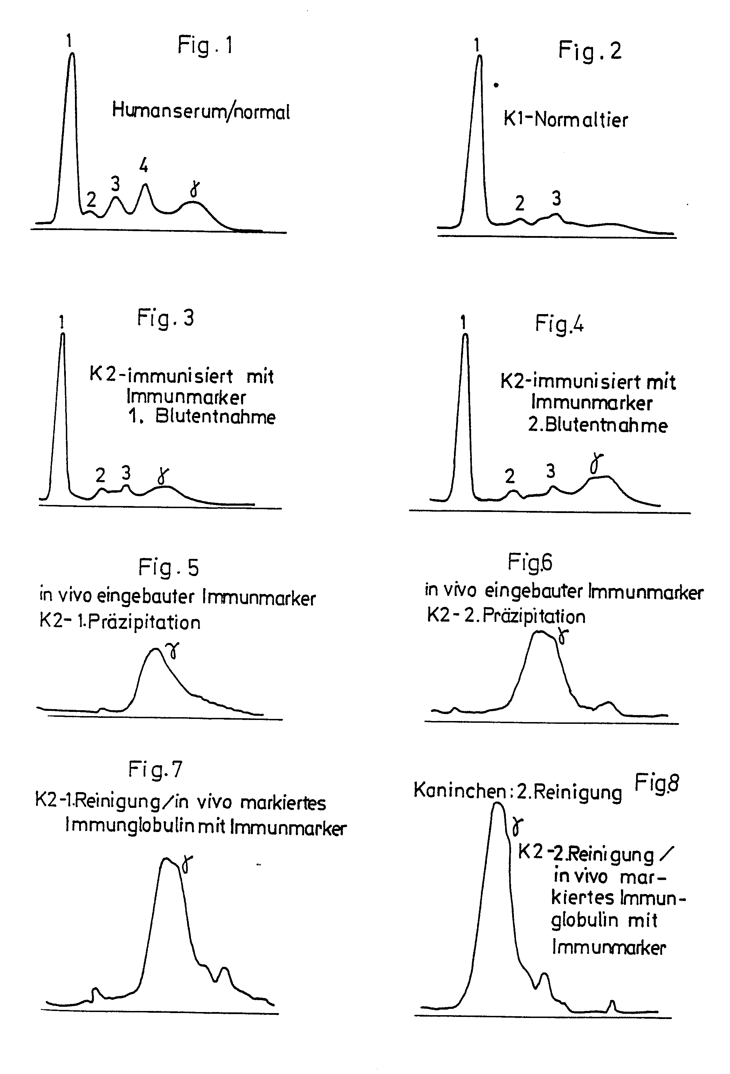

Die Figuren 1 bis 8 zeigen die Ergebnisse.

## Beispiel 16

Nachweis und Identifizierung von Viren in Flüssigkeiten.

Der angeregte Zustand zwischen einem fluoreszierenden Donatormolekül (Spendermolekül) und einem geeigneten fluoreszierenden Akzeptormolekül (Empfängermolekül) ist proportional zur Distanz zwischen beiden biofluorophoren Farbstoffen.

Dieses Phänomen wurde zur Virusidentifikation wie folgt genutzt:

virale Antikörper wurden getrennt an eine der zwei Komponenten oder an beide getrennt gekoppelt. Bei Vermischung beider mit Antiseren gekoppelten Komponenten trat kein Energie-Transfer ein. Erst bei Zugabe des Virus (Serumverdünnung oder Viruslösung (10μl Serum + 1000 μl Puffer) und durch die Wechselwirkung (interaction) einzelner Viruspartikel wurden Donatormoleküle und Akzeptormoleküle so eng aneinander geschoben (shifting), so daß diese starke Annäherung beider Molekülkomponenten eine Wirkungsgradsteigerung ergab, die den Energie-Transfer meßbar machte.

Die Kopplung von viralen Antiseren wurde bei pH 9.0 in wässriger Lösung durchgeführt.

Donatormoleküle und Akzeptormoleküle (Beispiel 2) wurden wie folgt angesetzt:

1 μg/ml biofluorophorer Farbmoleküle (Donatormoleküle) wurden in 0.15 mol/l $Na_2HPO_4 \bullet 2H_2O$ (pH-9.0) gelöst. 1 ml dieser Reaktionslösung auf 100 mg Protein wurden einer Immunglobulinlösung (Endlösung 10 mg/ml Protein) bei Raumtemperatur unter Rühren zugegeben. Der pH-Wert wurde durch Zugabe von 0.1 mol/l $Na_3PO_4 \bullet 10 H_2O$ unter Rühren auf 9.5 eingestellt. Die Lösung wurde unter leichtem Rühren 50 Min. bei Raumtemperatur aufbewahrt. Ein zweites Immunglobulin wurde wie folgt gekoppelt:

3 μg/ml biofluorophorer Farbmoleküle (Akzeptormoleküle) wurden in 0,15 mol/l NaCl und 10 % Vol. Carbonatpuffer-(5.8 ml einer 5,3 %igen $Na_2CO_3$-Lösung auf pH-9.5 einstellen) gelöst.

* ausgewaschenes Erythrozytengerüst

1 ml dieser Reaktionslösung auf 100 mg/ml Protein wurde einer zweiten Immunglobulinlösung unter Rühren zugeführt und 1 Std. im Eisbad bei 0°C zur Reaktion gebracht. Beide getrennt gekoppelten Reaktionslösungen wurden über PD-10-Säulen (Pharmacia) equilibriert mit 0.01 molar. PBS-Puffer, pH-7.5, gereinigt.

0.06 µg/ml Donatormolekül-Konjugat und 0.03 µg/ml Akzeptormolekül-Konjugat wurden in je 3 ml Messlösung vorbereitet und der Energie - Transfer am Perkin-Elmer-Spektralfluoromester gemessen.

Testversuche mit getrennt markierten Proteinen, ließen keine zusätzlichen unspezifischen Bindungen durch den Energie-Transfer bei fluoreszenzmarkierten Immunglobulinen erkennen. Der Virus-Antikörper-Komplex zeigte hohe Stabilität. Es wurden meßbare Verdünnungen bis zu sechs VAF-Moleküle (Virus-Antikörper-Farbstoff) erreicht.

Alle Reagenzien sind ausschließlich für die in vitro-Diagnostik bestimmt.

Originalverschlossen bleiben alle Reagenzien dieser IDT-Testkits bei 2° - 8°C bis zu dem auf der Testpackung angegebenen Verfallsdatum haltbar.

Wahlweise können je nach Testprinzip (Beispiele 9, 10, 13, 14) virale Antikörper, wie HIV I, II, III und noch weitere, bisher nicht isolierte HIV-Antikörper oder deren Antigene sowie virale Erkennungsproteine, Immunglobulinklassen und deren Untergruppen, Rezeptoren, Makrophagen, Phagozyten, Astrozyten oder spezifische monoklonale Antikörper oder MCA-Coctails (MCA = monoclonale Antikörper) getrennt an eine der zwei Farbstoff-Komponenten, oder an eine der Farbstoffmolekül-Komponenten kovalent gebunden werden.

Beispiel 17

13

Testkit für Immundeficiency-Test nach Beispiel 13

| Art der Reagenzien: | Packungsinhalt: |
|---|---|
| Mikrotiterplatte (Strips mit Rahmen) beschichtet mit Antigenen | 1 oder 10 Platten vakuumverpackt |
| 10fach konzentrierte Waschlösung Tris-Puffer,pH 7.4 mit 1% Tween 20 1 Teil Waschlsg + 9 Teile Aqua bidest. | 1 x 100 ml |
| Negative Kontrolle Humanserumserum negativ für alle Erreger, enthält 0.1% Natriumazid | 1 x 5 ml |
| Verdünnungsmittel für Proben(Konz.) 5% hochgereinigtes Molkenalbumin (Colostrum-lipidfrei) in 0.05 molar.PBS-Puffer,pH 7.2 und 0.1% Natriumazid gebrauchsfertige Verdünnungslösung 1 Teil Verd.Lsg. + 9 Teile Aqua bidest. | 1 x 50 ml |
| Fluoreszenzmarkiertes Anti-A,-D.-E,-M,-G | 5 x 5ml 0.03μg/ml Farbstoff |
| Fluoreszenzmarkierter HIV-Antikörper | 1 x 10 ml 0.03 μg/ml Farbstoff |
| Fluoreszenzmarkierte Erreger-Antikörper $IgG,IgM,HBs,IgG_1,IgG_2A,IgG_3,IgB$ | 7 x 5 ml 0.03 μg/ml Farbstoff |
| Protein-Molekular-Gewicht-Standard gebrauchsfertige Standards für die Bereiche picomol,femtomol,attomol, Ausgangsstandard = Nullpunkt | 9 x 2 ml |
| gebrauchsfertige 1 n NaOH | 1 x 15 ml |

Erläuterungen der Serum-Elektropheresegramme 1 - 8

Bild 1) Humanserum/Normal peak 1 = Albumin
peak 2 = $\alpha$1-Globulin
peak 3 = $\alpha$2-Globulin
peak 4 = $\beta$-Globulin
peak 5 = $\gamma$-Globulin

Bild 2) Kaninchenserum (K1) Normaltier peak 1 = Albumin
peak 2 = $\alpha$ -Globulin
peak 3 = $\beta$ -Globulin

Bild 3) Kaninchenserum (K2) immunisiert mit biofluorophoren Immunmarker

1.Blutentnahme nach 30 Tagen peak 1 = Albumin
peak 2 = $\alpha$ -Globulin
peak 3 = $\beta$ -Globulin
peak 4 = $\gamma$ -Globulin

Bild 4) Kaninchenserum (K2) immunisiert mit biofluorophoren Ummunmarker

2.Blutentnahme nach 45 Tagen peak 1 = Albumin
peak 2 = $\alpha$ -Globulin
peak 3 = $\beta$ -Globulin
peak 4 = $\gamma$ -Globulin

Bild 5) Kaninchenserum(K2) immunisiert mit biofluorophoren Immunmarker (gepooltes Vollbut aus 2 Blutentnahmen).

1.Präzipitation mit $Na_2SO_4$ gesättigt. peak 1 = unreines $\gamma$ -Globulin

Bild 6) Kaninchenserum (K2) immunisiert mit biofluorophoren Immunmarker (gepooltes Vollblut aus 2 Blutentnahmen).

5.Präzipitation mit $Na_2SO_4$ gesättigt. peak 1 = unreines $\gamma$ -Globulin mit deutlich besserer Darstellung

Bild 7) Kaninchenserum (K2) wie 5, 6)

1.Reinigung mit Sephadex G - 25 peak 1 = $\gamma$ -Globulin mit deutlich verstärktem peak. lks.u.re. des $\gamma$-peaks klenere peaks von Puffer-Restrückständen

Bild 8) Kaninschenserum (K2) wie 5,6,7)

2.Reinigung mit Stroma-Lyophilisat Peak 1 = $\gamma$ -Globulin, voll ausgeprägt rechtsseitige kleine peaks von Pufferlösg.

Ausbeute: in vivo fluoreszenzmarkiertes Gamma-Globulin

**Picomolbereich und Kalibrationskurve - Bombesin-Test mit normalem menschlichem Serum**

Extinktion = 0,148   =   14,83 pmol

  = 0,160   =   16,32 pmol

  = 0,169   =   18,49 pmol

  = 0,170   =   18,66 pmol

**Kalibrationskurve**

normales menschl. Serum - pmol/ml Bombesin          366 nm

| normales menschl. Serum - pmol/ml Bombesin | 366 nm | |
|---|---|---|
| 0 | 0,064 | |
| 3,78 | 0,082 | |
| 7,56 | 0,107 | |
| 15,13 | 0,151 | |
| 30,26 | 0,236 | Korrelationskoeffizient = 0,9997 |

Standardabweichung und Patientenseren zum Bombesin-Test

<u>Spektralphotometer UVIKON, Wellenlänge 366 nm/Luft</u>

Standard: normales menschliches Serum

Patientenseren

| | | |
|---|---|---|
| 0,126 | 0,12o | 0,134 |
| 0,133 | 0,127 | 0,122 |
| 0,131 | 0,128 | 0,125 |
| 0,128 | 0,127 | 0,127 |
| 0,130 | 0,128 | 0,128 |
| 0,126 | 0,133 | 0,123 |
| 0,127 | 0,12o | 0,126 |
| 0,129 | 0,133 | |

1) 0,137 = 13,90 picomol Bombesin

2) 0,133 = 13,08 picomol Bombesin

3) 0,186 = 23,92 picomol Bombesin

4) 0,132 = 12,88 picomol Bombesin

5) 0,138 = 14,10 picomol Bombesin

$\bar{x} \pm$ = SD 1,3

Mittel: 0,128 = 12,08 picomol

**Ansprüche**

1. Verfahren zur Herstellung eines Fluoreszenzfarbstoffes, dadurch **gekennzeichnet,** daß man Riboflavine, gelöst in Mercaptoethanol, mit Alphaliponsäure bei einem pH von etwa 8 umsetzt und nach Zugabe von Jodacetamid und Dyalisieren des Reaktionsgemisches destilliert und den Destillationsrückstand auf pH 5 bis 6 einstellt und diesen unter leichtem Erwärmen eine Reaktionslösung aus 1,4-Bis-(4-methyl-5-phenyl-2-oxazolyl)-benzol und p-Terphenyl in einem organischen Lösungsmittel, der Maleinsäureanhydrid zugesetzt ist, langsam zutropft und das Reaktionsgemisch nach mehrstündigem Stehen auf ein etwa neutrales pH einstellt und in üblicher Weise reinigt.

2. Verfahren nach Anspruch 1, dadurch **gekennzeichnet,** daß man bei der Umsetzung Riboflavine durch NAD, NADH oder NADP und die $\alpha$-Liponsäure durch andere optisch aktive Substanzen, welche S-S-Disulfidbrücken haben, ersetzt.

3. Fluoreszenzfarbstoff, hergestellt gemäß dem Verfahren nach Anspruch 1 oder 2.

4. Anwendung des Fluoreszenzfarbstoffes nach Anspruch 1 oder 2 zur Kopplung an Proteine, insbesondere Antiseren, zur Herstellung von markiertem Protein.

5. Verwendung des Farbstoffes nach Anspruch 1 oder 2 bzw. der farbstoffmarkierten Proteine für Fluoreszenzimmunoassay.

6. Verwendung des Farbstoffes nach Anspruch 1 oder 2 bzw. des an Proteine gekoppelten Farbstoffes zur Markierung von viralen Proteinen, Immunglobulinen, Zellkulturen, infektiösen Zellen, Viren und anderen Mikroorganismen, Klonen, Aszitesflüssigkeiten, monoclonalen Antikörpern und Antigenen für in vivo-Diagnostik und -Therapie.

7. Verwendung des Farbstoffes nach Anspruch 1 oder 2 zum Nachweis und zur Bestimmung von Tumormarkern oder zur Herstellung von Immunomarkern.

8. Kit für Immundeficiency-Test, bestend aus Mikrotiterplatte, beschichtet mit Antigenen, Waschlösung, Nagativkontrolle (Humanserum negativ für alle Erreger), 5%iges hochgereinigtes Molkenalbumin in PBS-Puffer als Verdünnungsmittel für Proben, fluoreszenzmarkiertes Anti-A,-D,-E,-M,-G, fluoreszenzmarkierte HIV-Antikörper, fluoreszenzmarkierte Erreger-Antikörper IgG, IgM, HBs, IgG$_1$, IgG$_2$A, IgG$_3$, IgB sowie Protein-Molekular-Gewicht-Standards für die Bereiche picomol, femtomol, und attomol und schließlich gegebenenfalls verdünnte NaOH.

9 Testkit zum Nachweis und zur Bestimmung von Tumormarkern, enthaltend myo-Inosit/Bombesin, PMGS-Standard, PBS-Puffergranulat f.pH 7,2, Trichloressigsäure 5N, Salzsäure 2N und NaOH 1,5 M.

Fig. 1

Humanserum/normal

Fig. 2

K1-Normaltier

Fig. 3

K2-immunisiert mit
Immunmarker
1. Blutentnahme

Fig. 4

K2-immunisiert mit
Immunmarker
2. Blutentnahme

Fig. 5

in vivo eingebauter Immunmarker
K2- 1. Präzipitation

Fig 6

in vivo eingebauter Immunmarker
K2- 2. Präzipitation

Fig. 7

K2-1. Reinigung/in vivo markiertes
Immunglobulin mit Immunmarker

Fig 8

Kaninchen: 2. Reinigung

K2-2. Reinigung /
in vivo markiertes Immunglobulin mit
Immunmarker

Verdünnungswerte / Bombesin-Test ( Kalibrationskurve)

Spektralphotometer UVIKON, Wellenlänge 366 nm

| | | |
|---|---|---|
| 0,102 | = | 7,56 picomol |
| 0,150 | = | 15,12 picomol |
| 0,214 | = | 30,25 picomol |

Fig. 9